(19) Europäisches Patentamt
European Patent Office
Office européen des brevets

(11) Publication number: **0 472 325 B1**

(12) # EUROPEAN PATENT SPECIFICATION

(45) Date of publication of patent specification: **28.12.94**

(51) Int. Cl.5: **C07D 213/40**, A61K 31/44

(21) Application number: **91307249.2**

(22) Date of filing: **07.08.91**

The file contains technical information submitted after the application was filed and not included in this specification

(54) **Acid addition salts of an optically active alaninanilide compound and pharmaceutical compositions containing the same.**

(30) Priority: **08.08.90 JP 211208/90**

(43) Date of publication of application:
**26.02.92 Bulletin 92/09**

(45) Publication of the grant of the patent:
**28.12.94 Bulletin 94/52**

(84) Designated Contracting States:
**AT BE CH DE ES FR GB IT LI NL SE**

(56) References cited:
**EP-A- 0 198 325**
**FR-A- 2 147 277**

(73) Proprietor: **Kissei Pharmaceutical Co., Ltd.**
**No. 19-48 Yoshino**
**Matsumoto-City**
**Nagano-Pref. 399-65 (JP)**

(72) Inventor: **Kamijo, Tetsuhide**
**No. 2525 Oaza Hirooka yoshida Shiojiri-shi**
**Nagano-ken (JP)**
Inventor: **Yamaguchi, Toshiaki**
**c/o Kissei Daini-Seiyuryo,**
**No 1-2-34 Nomizo Mokko**
**Matsumoto-shi,**
**Nagano-ken (JP)**
Inventor: **Ikegami, Kazuhiko**
**No 3176 Oazo Yutaka,**
**Misato-mura**
**Minamiazumi-gun,**
**Nagano-ken (JP)**

(74) Representative: **Baverstock, Michael George Douglas et al**
**BOULT, WADE & TENNANT**
**27 Furnival Street**
**London, EC4A 1PO (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to acid addition salts of an optically active alaninanilide compound.

A pharmacologically active alaninanilide compound having an asymmetic carbon atom has been used in the form of a racemic mixture as an active ingredient. More recently an optically active compound of the pharmacological active compound has been preferably used as an active ingredient to avoid side effects.

U.S. Patent No. 4,696,930 discloses substituted aniline compounds represented by the formula:

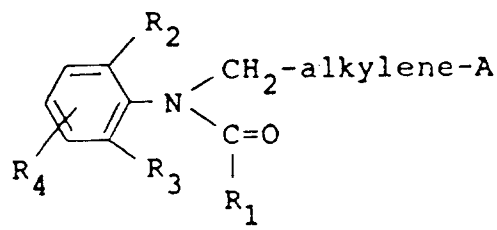

(III)

wherein $R_1$ is alkylene'-$NH_2$ or alkylene'-A'; $R_2$, $R_3$ and $R_4$ are, independently, a hydrogen atom or a methyl group; A and A' are independently, an unsubstituted or a lower alkyl or an aryl substituted pyridinyl, or pyrimidinyl group; alkylene and alkylene' are independently, straight chain alkylene moieties of 1-5 carbon atoms optionally substituted with one or more alkyl substituents of 1-5 carbon atoms, or when an asymmetric carbon is present, an enantiomer thereof, or a racemic mixture thereof; or a pharmaceutically acceptable salt thereof being useful as antiarrhythmic agents.

In that document, rac.-2-amino-N-(2,6-dimethylphenyl)-N-[3-(3-pyridyl)propyl]propanamide represented by the formula:

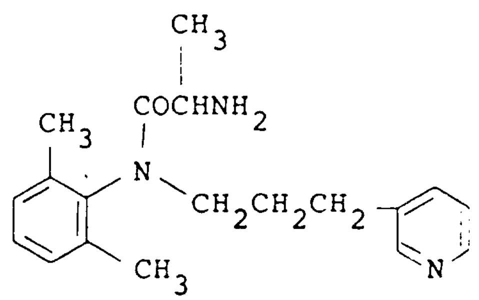

(IV)

which is closely related to the salts of the present invention is actually described. However, the compound of the formula (IV) above is a racemate not an optically active compound.

An optically pure alaninanilide compound in the free form of formula (IV) is an oily substance, therefore it is very hard to formulate such an optically pure compound in a pharmaceutical composition.

Furthermore, a hydrochloric acid salt thereof has a highly hygroscopic property, and the water contained in such a salt varies depending upon the moisture in atmosphere in which that salt is allowed to stand. In an atmosphere of high humidity, such a salt is deliquesced.

Accordingly, the net weight of that salt tends to vary due to its hygroscopic property of absorbing moisture from atmosphere. Taking into consideration the fact that a pharmaceutical composition has always to contain an active agent at a prescribed ratio so as to achieve the expected effect and safety, such a salt is not necessarily desirable for preparing pharmaceutical compositions.

FR-A-2 147 277 describes acid addition salts, such as tartaric salts, of 2-amino-2',6'-propionoxylidide having antiarrhythmic properties.

The present invention provides an acid addition salt of an optically active alaninanilide compound of formula (I) below useful for drugs.

The present invention further provides a pharmaceutical composition containing a salt of an optically active alaninanilide compound as an active ingredient.

In accordance with the present invention there is provided an acid addition salt of an optically active alaninanilide compound represented by the formula:

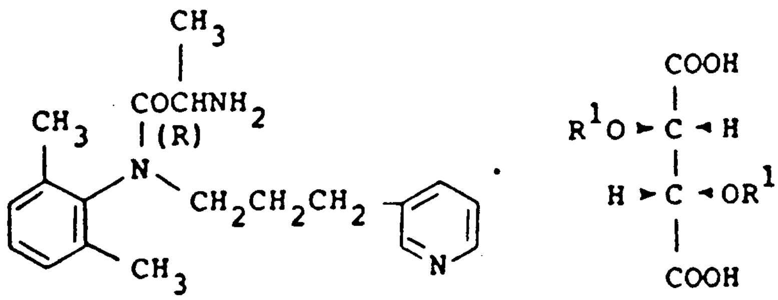

(I)

wherein $R^1$ is a hydrogen atom, a benzoyl group or a toluoyl group; and the carbon atom marked with (R) means a carbon atom in the R-configuration.

Our extensive research and experimentation has shown that the salts of the formula (I) above absolutely solve the problems of the prior art compound and salts. That is, the salts of the formula (I) are non-hygroscopic and thus have a constant net weight and are stable without being affected by moisture contained in the atmosphere. They are stable in an atmosphere having about 50% relative humidity at about 25-26 °C. They can thus advantageously be used to formulate pharmaceutical compositions which can be of high quality.

The acid addition salts of an optical active alaninanilide compound represented by formula (I) of the present invention exhibit anti-arrhythmic activities, thromboxane $A_2$-synthetase inhibitory activities and platelet aggregation inhibitory activities in the same way as does a racemate of formula (IV) or an acid addition salt thereof. Thus they are useful as drugs for the treatment of arrhythmias, thrombosises or ischemic cardiac insufficiencies.

The acid addition salts of formula (I) of the present invention can be prepared by treating a free amine of an optically active alaninanilide compound or a racemate with an optical active tartaric acid derivative such as D-tartaric acid, dibenzoyl-D-tartaric acid, or ditoluoyl-D-tartaric acid, and then, if necessary, by fractional crystallization of the obtained crude acid addition salt in accordance with the usual manner.

The compound of formula (IV) can be prepared in a similar way to that described in the aforementioned U.S. Patent 4,696,930.

For example, N-carboethoxyphthalimide is reacted with alanine to obtain 2-phthalimidopropionic acid, and the reaction product is converted to a reactive functional derivative thereof, such as acid chloride. The acid chloride is then reacted with N-[3-(3-pyridyl)-propyl]-2,6-dimethylaniline to obtain 2-phthalimido-N-(2,6-dimethylphenyl)-N-[3-(3-pyridyl)propyl]-propanamide and then the product is treated with a base, such as methylamine to prepare the desired product of the formula (IV).

In the preparation of the compound of formula (IV) described above, if the optically active D-alanine is employed as a starting material, a desired optically active compound can be obtained in high purity. If that prepared compound is then employed as the starting material to prepare an acid addition salt of formula (I), the desired acid addition salt can be purified from the obtained crude acid addition salt by a very simple purification manner.

In the present invention the acid addition salt, (R)-2-amino-N-(2,6-dimethylphenyl)-N-[3-(3-pyridyl)-propyl]-propanamide D-tartrate represented by the formula (II):

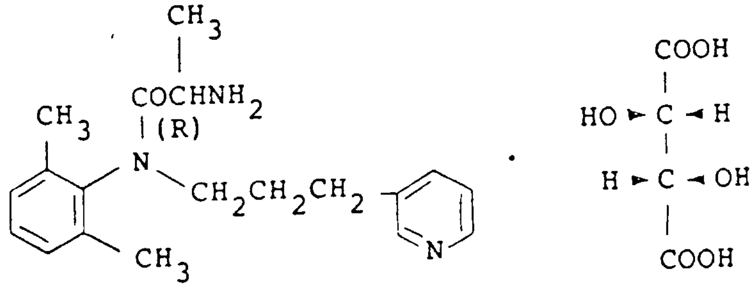

(II)

wherein the carbon atom marked with (R) has the same meaning as described above, is the most preferred.

When the acid addition salts of formula (I) of the present invention are employed in the treatment of a patient, any pharmaceutical compositions containing the compound as an active ingredient, such as tablets, capsules, powders, granules, syrups and injectable preparations, may be formulated and may be administered orally or parenterally. These pharmaceutical compositions can be formulated in accordance with usual formulation manners.

The dosage of the compound may be determined in accordance with sex, age and body weight of a patient, the nature of the disease and the degree of illness. It may, for example, be 1-100 mg per day in oral administration in the adult and 0.1-1000 mg per day in parenteral administration in the adult.

The present invention is further illustrated by way of the following Examples and Reference Examples. The melting points of the products obtained were uncorrected. The optical purities of the products were calculated by chiral HPLC [Column: SUMICHIRAL OA-4600 (Sumitomo Chemical Co., Ltd.) 5 µm, 4 mm i.d. x 25 cm; Eluent: n-hexane : dichloroethane : ethanol = 80:15:5; Flow rate: 1.0 ml/min; Detection: UV 260 nm]. Samples were treated with acetic anhydride to derive acetates thereof prior to the chromatography.

Reference Example 1

(R)-2-Phthalimidopropionic acid

To a solution of 50 g of D-alanine in 500 ml of water, was added 78 ml of triethylamine, and 123 g of N-carbethoxyphthalimide was added to the mixture in small portions under ice-cooling. The mixture was stirred for the following 30 minutes. The precipitated materials were filtered off and the filtrate was acidified to pH 1-2 with 6N-hydrochloric acid and was cooled to 5°C to precipitate crystals. The crystals were collected by filtration and were washed twice each time with 100 ml of water and dried at 40°C under reduced pressure to give 93 g of (R)-2-phthalimidopropionic acid as white crystals.

Yield: 77%

Melting Point: 148-149°C

Specific rotation: $[\alpha]_D^{25}$ = +26.8°

(c = 1.044, MeOH)

Reference Example 2

(R)-2-Phthalimido-N-(2,6-dimethylphenyl)-N-[3-(3-pyridyl)propyl]propanamide hydrochloride

To a suspension of 10 g of (R)-2-phthalimido-propionic acid in 10 ml of thionyl chloride was added 1.8 ml of dry dimethylformamide and the mixture was stirred for 5 hours at room temperature. The solvent was evaporated in vacuo and 50 ml of dry dichloroethane were added to the residue and the mixture was concentrated in vacuo. The same procedure was repeated twice to remove excess thionyl chloride completely and to obtain an acid chloride.

The obtained acid chloride was dissolved in 50 ml of dry dichloroethane, and a solution of 9.97 g of N-[3-(3-pyridyl)propyl]-2,6-dimethylaniline in 5 ml of dry dichloroethane was dropped into the solution while keeping the temperature of the solution at 3-5°C. Following this the reaction mixture was stirred at room temperature over-night.

The precipitates were filtered off and washed twice each time with 50 ml of dry dichloroethane, the filtrate was concentrated in vacuo to give 22.6 g of (R)-2-phthalimido-N-(2,6-dimethylphenyl)-N-[3-(3-pyridyl)-propyl]propanamide hydrochloride as an amorphous powder.

Optical purity: 98% ee

IR (KBr): $\nu$co 1715, 1660 $cm^{-1}$

Reference Example 3

(R)-2-Amino-N-(2,6-dimethylphenyl)-N-[3-(3-pyridyl)propyl]propanamide

To a solution of 22.6 g of (R)-2-phthalimido-N-(2,6-dimethylphenyl)-N-[3-(3-pyridyl)propyl]-propanamide hydrochloride obtained in the Reference Example 2 in 113 ml of methanol was added 82 ml of 40% methylamine methanol solution and the reaction mixture was stirred overnight. The solvent was evaporated in vacuo, the residue was dissolved in 100 ml of 2N-hydrochloric acid and the solution was washed twice each time with 70 ml of methylenechloride. The aqueous layer was neutralized to pH 8 with sodium bicarbonate and the solution was extracted 3 times each time with 70 ml of methylene chloride.

The organic layer was washed with water and dried over anhydrous sodium sulfate and the solvent was evaporated in vacuo to give 8.4 g of (R)-2-amino-N-(2,6-dimethylphenyl)-N-[3-(3-pyridyl)propyl]-propanamide.

Optical purity: 98% ee
IR (neat): $\nu$co 1650 $cm^{-1}$

Example 1

(R)-2-Amino-N-(2,6-dimethylphenyl)-N-[3-(3-pyridyl)propyl]propanamide D-tartrate

A mixture of 8.4 g of (R)-2-amino-N-(2,6-dimethylphenyl)-N-[3-(3-pyridyl)propyl]propanamide and 3.2 g of D-tartaric acid was dissolved in 33.5 ml of 3% aqueous ethanol and an authentic sample was seeded to the solution and the solution was allowed to stand overnight at room temperature.

The precipitated crystals were collected by filtration and dried under reduced pressure at 60 °C to give 8.2g of (R)-2-amino-N-(2,6-dimethylphenyl)-N-[3-(3-pyridyl)propyl]propanamide D-tartrate as white crystals.

Optical purity: 100% ee
Melting point: 150-151 °C
Specific rotation: $[\alpha]_D^{20}$ = -58.7 °
(c = 1.000, $H_2O$)

Example 2

(R)-2-Amino-N-(2,6-dimethylphenyl)-N-[3-(3-pyridyl)propyl]propanamide dibenzoyl-D-tartrate

A mixture of 10 g of (RS)-2-amino-N-(2,6-dimethylphenyl)-N-[3-(3-pyridyl)propyl]propanamide and 11.5 g of dibenzoyl D-tartaric acid were dissolved in 100 ml of ethanol and an authentic sample was seeded to the solution and the solution was allowed to stand overnight at 60 °C. The precipitated crystals were collected by filtration and dried to give 9.92 g of (R)-2-amino-N-(2,6-dimethylphenyl)-N-[3-(3-pyridyl)propyl]-propanamide dibenzoyl D-tartrate.

Yield: 46.1%; Optical purity: 92.6% ee

The crystals were dissolved in a mixed solvent of 100 ml of chloroform and 200 ml of ethanol and the solution was heated under reflux to remove chloroform completely. Ethanol was added to the residual mixture to prepare an ethanol solution of about 200 ml in volume and the solution was allowed to stand overnight at 60 °C. The precipitated crystals were collected by filtration and dried to give 8,18 g of (R)-2-amino-N-(2,6-dimethylphenyl)-N-[3-(3-pyridyl)propyl]propanamide dibenzoyl D-tartrate.

Yield: 38.1%
Optical purity: 100% ee
Melting point: 166-167 °C
Specific rotation: $[\alpha]_D^{25}$ = +38.7 °
(c = 1.005, MeOH)

Example 3

(R)-2-Amino-N-(2,6-dimethylphenyl)-N-[3-(3-pyridyl)propyl]propanamide D-tartrate

50 ml of a saturated agueous sodium bicarbonate solution was added to 8.18 g of (R)-2-amino-N-(2,6-dimethylphenyl)-N-[3-(3-pyridyl)propyl]propanamide dibenzoyl D-tartrate (optical purity: 100% ee) obtained in Example 2 and the mixture was extracted three times with 50 ml, 20 ml and 20 ml of methylene chloride.

The organic layer was washed successively with a saturated aqueous sodium bicarbonate solution and water and was dried over anhydrous sodium sulfate. The solvent was evaporated in vacuo to give 3.90 g of (R)-2-amino-N-(2,6-dimethylphenyl)-N-[3-(3-pyridyl)propyl]propanamide.

A mixture of 3.90 g of the free amine obtained above and 1.86 g of D-tartaric acid was dissolved in 15 ml of 3% aqueous ethanol and an authentic sample was seeded to the solution and the solution was allowed to stand overnight at room temperature.

The precipitated crystals were collected by filtration and dried to give 5.84 g of (R)-2-amino-N-(2,6-dimethylphenyl)-N-[3-(3-pyridyl)propyl]propanamide D-tartrate. The melting points and optical rotation of the product were identical to that of the compound prepared in Example 1.

Example 4

(R)-2-Amino-N-(2,6-dimethylphenyl)-N-[3-(3-pyridyl)propyl]propanamide di-p-toluoyl D-tartrate

A mixture of 500 mg of (R)-2-amino-N-(2,6-dimethylphenyl)-N-[3-(3-pyridyl)propyl)propanamide and 620 mg of di-p-toluoyl D-tartaric acid was dissolved in 5 ml of isopropanol and an authentic sample was seeded to the solution and the solution was allowed to stand overnight at room temperature. The precipitated crystals were collected by filtration and dried at 60 °C to give 608 mg of (R)-2-amino-N-(2,6-dimethylphenyl)-N-[3-(3-pyridyl)propyl]propanamide di-p-toluoyl D-tartrate as white crystals.

Optical purity: 100% ee

Melting point: 126-128 °C

Specific rotation: $[\alpha]_D^{25}$ = +62.8 °

(c = 1.031, MeOH)

Example 5

Hygroscopicities determination test

The hygroscopicities of (R)-2-amino-N-(2,6-dimethylphenyl)-N-[3-(3-pyridyl)propyl]propanamide hydrochloride (hereinafter referred to as hydrochloride of the R-form compound); D-tartrate, dibenzoyl-D-tartrate and di-p-toluoyl D-tartrate of (R)-2-amino-N-(2,6-dimethylphenyl)-N-[3-(3-pyridyl)propyl]propanamide (hereinafter referred to as D-tartrate of the R-form compound, dibenzoyl D-tartrate of the R-form compound and di-p-toluoyl D-tartrate of the R-form compound respectively), were determined as follows.

Each sample was weighed out in about 0.1 g or 0.2 g into a weighing dish having a diameter of about 2.7-3.0 cm.

The samples were allowed to stand in a constant moisturized vessel containing a saturated aqueous calcium nitrate solution and having 51% of relative humidity at about 25-26 °C.

The change of weight on standing of each sample was measured and the hygroscopicity of each sample was determined according to the following equation.

$$\text{Hygroscopicity (\%)} = \frac{W_3 - W_2}{W_2 - W_1} \times 100$$

$W_1$: Weight of the empty weighing dish (g)

$W_2$: Weight of the weighing dish containing a sample before standing (g)

$W_3$: Weight of the weighing dish containing a sample after standing (g)

Hygroscopicity (%) of D-tartrate of the R-form compound (Sample B) and dibenzoyl D-tartrate of the R-form compound (Sample C) and di-p-toluoyl D-tartrate of the R-form compound (Sample D) in comparison with that of hydrochloride of the R-form compound (Sample A) is given below.

| Standing time (hr) | Sample A (%) | Sample B (%) | Sample C (%) | Sample D (%) |
|---|---|---|---|---|
| 0 | 0.00 | 0.00 | 0.00 | 0.00 |
| 0.5 | 4.39 | - | - | - |
| 1 | 5.77* | 0.89 | 0.13 | 0.61 |
| 2 | 7.54* | - | - | - |
| 3 | - | 1.16 | 0.17 | 0.95 |
| 5 | - | 1.25 | - | - |
| 6 | 8.51* | - | 0.18 | 1.13 |
| 9 | | 1.33 | | |

* means deliquescent

## Claims

### Claims for the following contracting States : GB, FR, DE, CH, LI NL, IT, SE, BE, AT

**1.** An acid addition salt of an optically active alaninanilide compound represented by the formula (I):

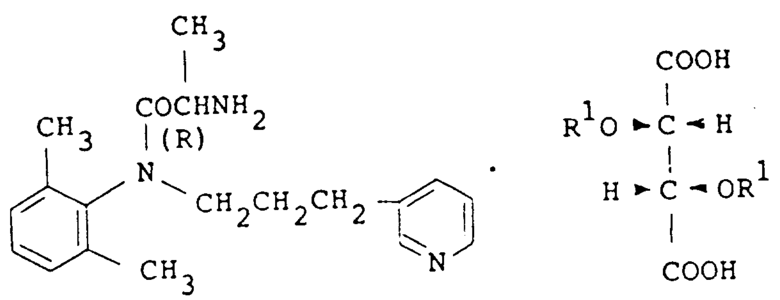

(I)

wherein $R^1$ is a hydrogen atom, a benzoyl group or a toluoyl group; and the carbon atom marked with (R) means a carbon atom in a form of R-configuration.

**2.** An acid addition salt of an optically active alaninanilide compound as claimed in claim 1, represented by the formula (II):

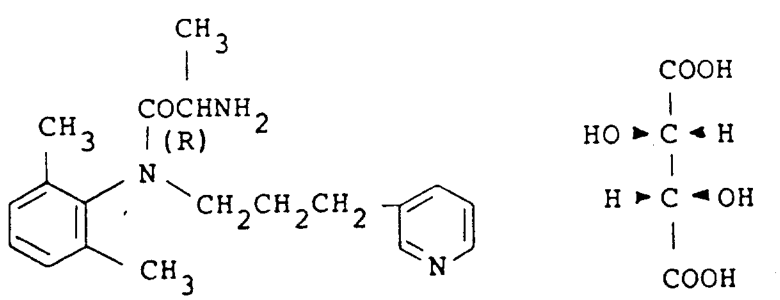

(II)

wherein the carbon atom marked with (R) has the same meaning mentioned above.

**3.** A pharmaceutical composition for the treatment or arrhythmias, thromboses or ischemic cardiac insufficiencies in a mammal, which composition contains an effective dosage of an acid addition salt of an optically active of an alaninanilide compound corresponding to the formula represented in claim 1 or claim 2.

4. Use of an acid addition salt of an optically active alaninanilide compound corresponding to the formula represented in claim 1 or claim 2 in the preparation of a medicament for the treatment of arrhythmias, thromboses or ischemic cardiac insufficiencies in a mammal.

**Claims for the following Contracting State : ES**

1. A method of preparing an acid addition salt of an optically active alaninanilide compound represented by the formula (I):

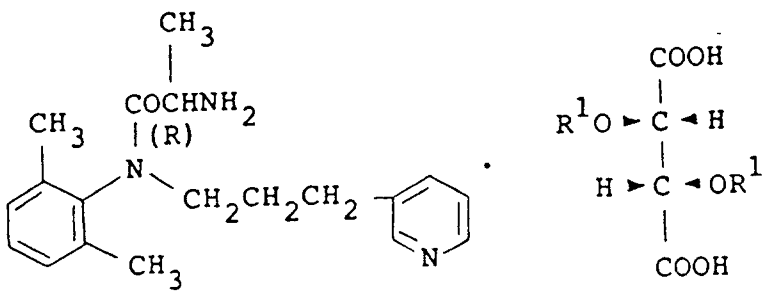

(I)

wherein $R^1$ is a hydrogen atom, a benzoyl group or a toluoyl group, and the carbon atom marked with (R) means a carbon atom in a form of R-configuration, which comprises reacting a free amine of an optically active alaninanilide compound of formula (V)

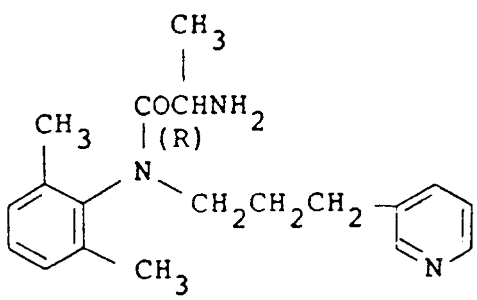

with an optically active tartaric acid derivative selected from D-tartaric acid, dibenzoyl-D-tartaric acid and ditoluoyl-D-tartaric acid.

2. A method of preparing an acid addition salt of an optically active alaninanilide compound represented by the formula (I):

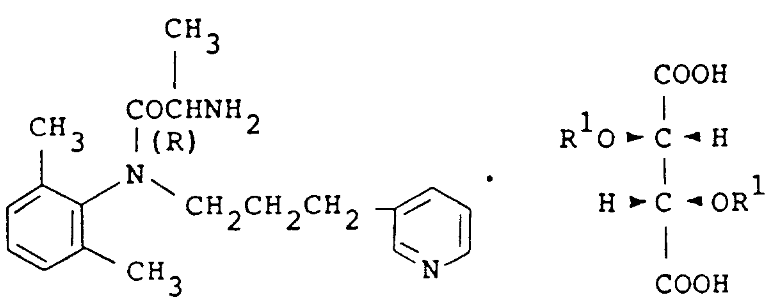

(I)

wherein $R^1$ is a hydrogen atom, a benzoyl group or a toluoyl group, and the carbon atom marked with (R) means a carbon atom in a form of R-configuration, which comprises reacting a free amine of a racemate of an alaninanilide compound of formula (IV)

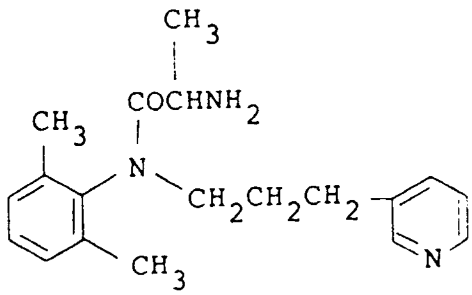

(IV)

with an optically active tartaric acid derivative selected from D-tartaric acid, dibenzoyl-D-tartaric acid and ditoluoyl-D-tartaric acid, followed by fractional crystallization of the obtained crude acid addition salt to yield the desired optically active salt.

**3.** A method as claimed in claim 1 or claim 2 in which $R^1$ is a hydrogen atom.

**4.** A method as claimed in any one of claims 1 to 3 in which the reaction is carried out in ethanol, aqueous ethanol, or isopropanol.

**Patentansprüche**
**Patentansprüche für folgende Vertragsstaaten : GB, FR, DE, CH, LI NL, IT, SE, BE, AT**

**1.** Säureadditionssalz einer optisch aktiven Alaninanilid-Verbindung der Formel (I):

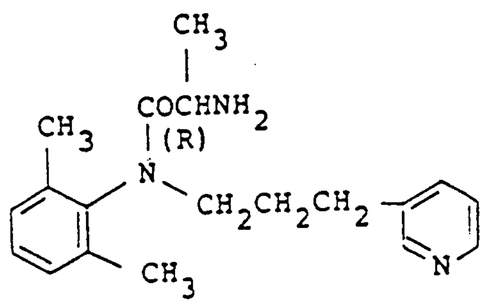

·

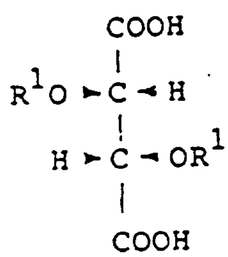

(I)

worin $R^1$ ein Wasserstoffatom, eine Benzoylgruppe oder eine Toluoylgruppe darstellt; und das mit (R) bezeichnete Kohlenstoffatom ein Kohlenstoffatom in einer Form der R-Konfiguration bedeutet.

**2.** Säureadditionssalz einer optisch aktiven Alaninanilid-Verbindung nach Anspruch 1, mit der Formel (II):

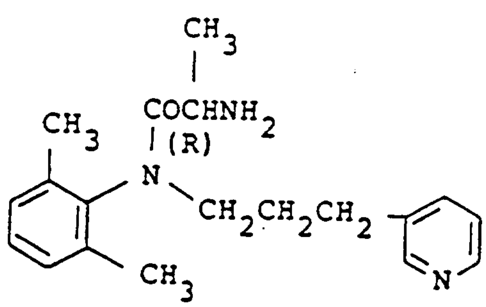

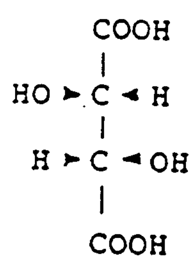

(II)

worin das mit (R) bezeichnete Kohlenstoffatom die gleiche Bedeutung wie oben hat.

**3.** Pharmazeutische Zubereitung zur Behandlung von Arrhythmien, Thrombosen oder ischämischen Herzinsuffizienzen bei einem Säuger, wobei die Zubereitung eine wirksame Dosis eines Säureadditionssalzes einer optisch aktiven Alaninanilid-Verbindung entsprechend der Formel von Anspruch 1 oder 2 enthält.

4. Verwendung eines Saureadditionssalzes einer optisch aktiven Alaninanilidverbindung entsprechend der Formel von Anspruch 1 oder 2, zur Herstellung eines Medikaments für die Behandlung von Arrhythmien, Thrombosen oder ischämischen Herzinsuffizienzen bei einem Säuger.

**Patentansprüche für folgenden Vertragsstaat : ES**

1. Verfahren zur Herstellung eines Säureadditionssalzes, einer optisch aktiven Alaninanilid-Verbindung der Formel (I):

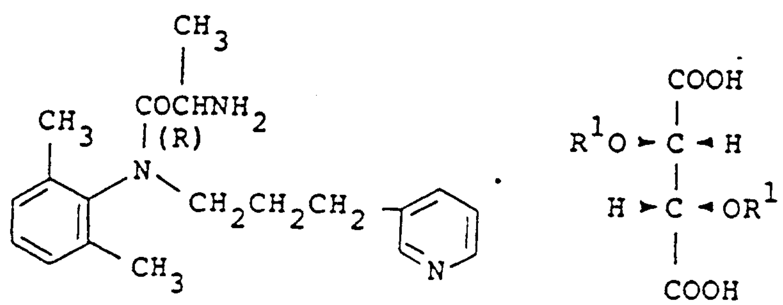

(I)

worin $R^1$ ein Wasserstoffatom, eine Benzoylgruppe oder eine Toluoylgruppe darstellt; und das mit (R) bezeichnete Kohlenstoffatom ein Kohlenstoffatom in einer Form der R-Konfiguration bedeutet; welches die Umsetzung eines freien Amins einer optisch aktiven Alaninanilid-Verbindung der Formel (V)

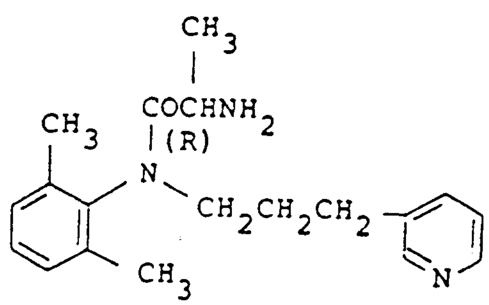

mit einem optisch aktiven Weinsäurederivat, ausgewählt aus D-Weinsäure, Dibenzoyl-D-Weinsäure und Ditoluoyl-D-Weinsäure umfaßt.

2. Verfahren zur Herstellung eines Säureadditionssalzes einer optisch aktiven Alaninanilidverbindung der Formel (I):

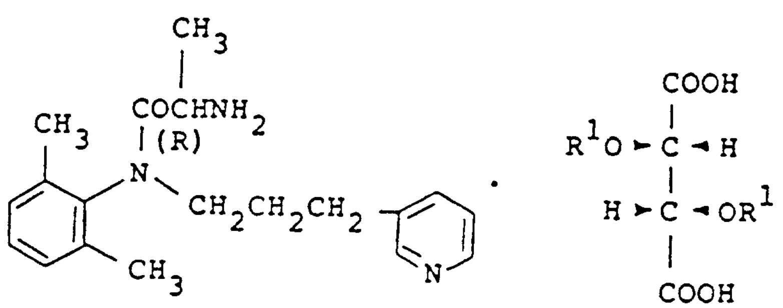

(I)

worin $R^1$ ein Wasserstoffatom, eine Benzoylgruppe oder eine Toluoylgruppe darstellt; und das mit (R) bezeichnete Kohlenstoffatom ein Kohlenstoffatom in einer Form der R-Konfiguration bedeutet; welches die Umsetzung eines freien Amin eines Racemats einer Alaninanilid-Verbindung der Formel (IV)

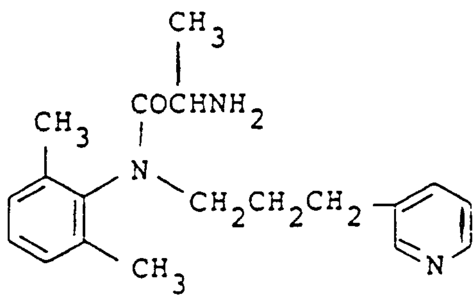

(IV)

mit einem optisch aktiven Weinsäurederivat, ausgewählt aus D-Weinsäure, Dibenzoyl-D-Weinsäure und Ditoluoyl-D-Weinsäure, und die anschließende fraktionierte Kristallisation des erhaltenen rohen Säureadditionssalzes zur Gewinnung des gewünschten optisch aktiven Salzes umfaßt.

**3.** Verfahren nach Anspruch 1 oder 2, worin $R^1$ ein Wasserstoffatom darstellt.

**4.** Verfahren nach einem der Ansprüche 1 bis 3, worin die Reaktion in Ethanol, wäßrigem Ethanol oder Isopropanol ausgeführt wird.

**Revendications**

**Revendications pour les Etats contractants suivants : GB, FR, DE, CH, LI, NL, IT, SE, BE, AT**

**1.** Sel d'addition d'acide d'un composé d'alaninanilide optiquement actif représenté par la formule (I):

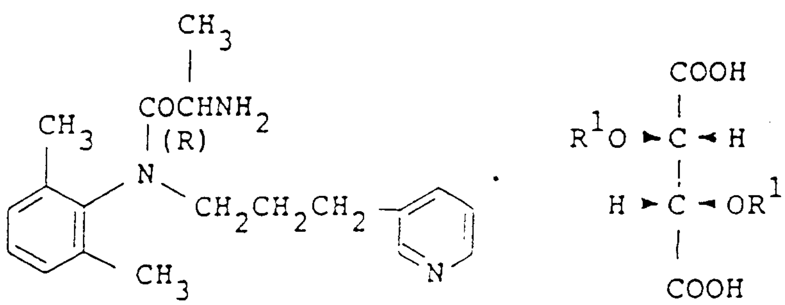

(I)

dans laquelle $R^1$ représente un atome d'hydrogène, un groupe benzoyle ou un groupe toluoyle et l'atome de carbone marqué par (R) désigne un atome de carbone sous la forme de configuration R.

**2.** Sel d'addition d'acide d'un composé d'alaninanilide optiquement actif suivant la revendication 1, représenté par la formule (II) :

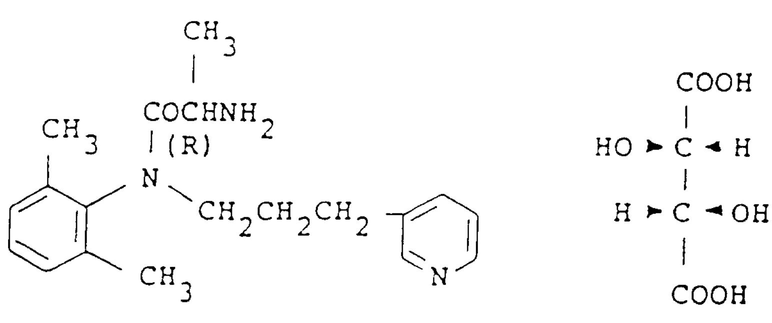

(II)

dans laquelle l'atome de carbone marqué par (R) a la même signification que mentionnée ci-dessus.

**3.** Composition pharmaceutique pour le traitement d'arythmies, de thromboses ou d'insuffisances cardiaques ischémiques chez un mammifère, laquelle composition contient un dosage efficace d'un sel d'addition d'acide d'un composé d'alaninanilide optiquement actif correspondant à la formule représentée dans la revendication 1 ou la revendication 2.

**4.** Utilisation d'un sel d'addition d'acide d'un composé d'alaninanilide optiquement actif correspondant à la formule représentée dans la revendication 1 ou la revendication 2 dans la préparation d'un médicament pour le traitement d'arythmies, de thromboses ou d'insuffisances cardiaques ischémiques chez un mammifère.

**Revendications pour l'Etat contractant suivant : ES**

**1.** Procédé de préparation d'un sel d'addition d'acide d'un composé d'alaninanilide optiquement actif représenté par la formule (I) :

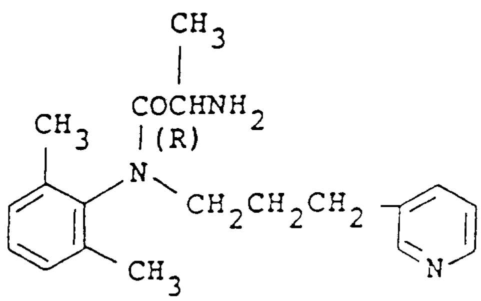 · 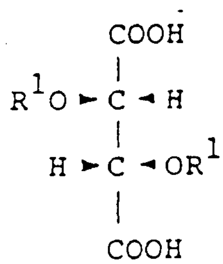 (I)

dans laquelle R[1] représente un atome d'hydrogène, un groupe benzoyle ou un groupe toluoyle et l'atome de carbone marqué par (R) désigne un atome de carbone sous la forme de configuration R, qui comprend la réaction d'une amine libre d'un composé d'alaninanilide optiquement actif de formule (V) :

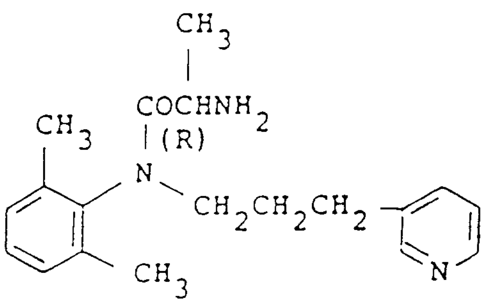

avec un dérivé d'acide tartrique optiquement actif choisi parmi l'acide D-tartrique, l'acide dibenzoyl-D-tartrique et l'acide ditoluoyl-D-tartrique.

**2.** Procédé de préparation d'un sel d'addition d'acide d'un composé d'alaninanilide optiquement actif représenté par la formule (I) :

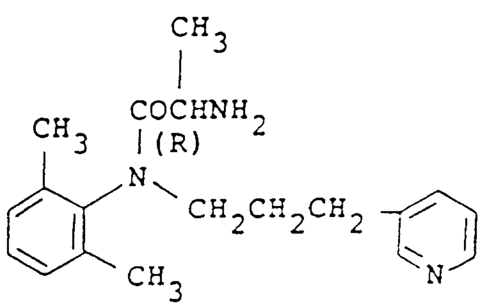 · 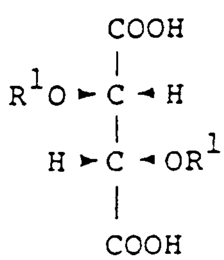 (I)

dans laquelle R[1] représente un atome d'hydrogène, un groupe benzoyle ou un groupe toluoyle et l'atome de carbone marqué par (R) désigne un atome de carbone sous la forme de configuration R, qui comprend la reaction d'une amine libre d'un racémate d'un composé d'alininanilide de formule (IV) :

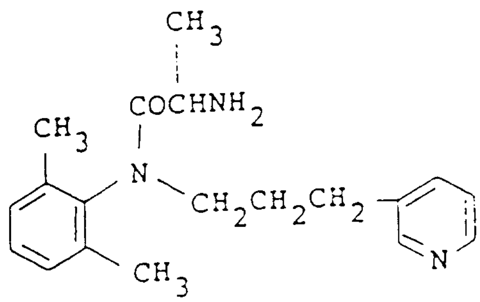

(IV)

avec un dérivé d'acide tartrique optiquement actif choisi parmi l'acide D-tartrique, l'acide dibenzoyl-D-tartrique et l'acide ditoluoyl-D-tartrique, suivie d'une cristallisation fractionnée du sel d'addition d'acide brut obtenu pour donner le sel optiquement actif désiré.

3. Procédé suivant l'une ou l'autre des revendications 1 et 2, dans lequel $R^1$ représente un atome d'hydrogène.

4. Procédé suivant l'une quelconque des revendications 1 à 3, dans lequel la réaction est réalisée dans de l'éthanol, de l'éthanol aqueux ou de l'isopropanol.